# EUROPEAN PATENT APPLICATION

(11) **EP 3 067 116 A1**
(43) Date of publication of application: **14.09.2016**
(21) Application number: 15382114.5
(22) Date of filing: 13.03.2015
(51) Int. Cl.: B01L 3/00, G01N 33/543, G01N 35/00

(54) **Device for electrochemical detection with magnetic particles**

(71) Applicant: Fundació Hospital Universitari Vall d'Hebron - Institut de Recerca, 08035 Barcelona (ES); Consejo Superior de Investigaciones Cientificas (CSIC), 28006 Madrid (ES)
(72) Inventor: Baldrich Rubio, Eva, 08221 TERRASSA (ES); Del Campo García, Javier, 28006 MADRID (ES)
(74) Representative: ZBM Patents - Zea, Barlocci & Markvardsen

(57) **Abstract**

The device (100) comprises a base component (10) for supporting at least one electrode (30), a cover component (60) for at least partially covering the base component (10) and having at least one opening (65) to confine a volume of a solution and for feeding magnetic particles to the electrode (30) for electrochemical detection; and at least one magnet (50) in at least one magnet support (40) that can be moved at least in two different positions for switching on/off the magnetic field to the electrodes (30) for confining or releasing the magnetic particles.

## Description

Devices and methods for electrochemical detection with magnetic particles are disclosed herein.

The present devices and methods can be used in general in bioassays, for example those based on affinity capture of an analyte using a specific bioreceptor and/or ligand attached to magnetic particles, but they may be also efficiently used in electrostatic capture of molecules such as drugs or neurotransmitters employing particles with surface charges or other types of molecular interactions. The present devices and methods can be applied to a wide range of applications.

### BACKGROUND

The use of magnetic particles for selective isolation, separation and purification processes is known and widely used, for example in bioassays. Magnetic separation requires the use of simple equipment and provides more efficient and faster assays than affinity capture using two-dimensional structures, such as in enzyme-linked immunosorbent assay (ELISA). It has been also shown that magnetic particle based assays allow detection limits to be improved compared to classical ELISA.

In magnetic particle based assays, magnetic particles are modified with bioreceptors, ligands, molecules, functional or charged groups, so that they bind specifically a selected target, such as a biomolecule, drug, neurotransmitter, cell or microorganism. These modified magnetic particles are then brought in contact with a sample; hence, in the presence of the target of interest, this is specifically bound and subsequently isolated, separated, purified and/or concentrated by applying a magnetic field, for example through the use of permanent magnets. Once isolated, the molecular target is detected by different means. For instance, magnetic particle-bound microorganisms can be culture growth; electroactive molecules and compounds can be detected electrochemically; fluorescent and coloured molecules and compounds can be detected spectrophotometrically. Otherwise, most molecular targets can be detected in sandwich assay formats, using a second bioreceptor/ligand modified with an appropriate label

(e.g.: an enzyme, fluorophore, electroactive label, nanomaterial, etc).

A number of magnetic electrochemical immunoassays, e.g. for the detection of diagnostic biomarkers, have been disclosed in the art. Some of these magnetic electrochemical immunoassays are based on the use of carbon paste electrodes (CPEs). CPEs consist of a cylinder body having an electrical connector and, optionally, an embedded permanent magnet. The cylinder body is filled with a conducting paste which acts as the working electrode. In the case of magnetic electrochemical immunoassays, magnetic particle bound analytes are then irreversibly confined onto the electrode surface by the action of the embedded magnet. Hence, once the detection process has been carried out, the magnetic particles have to be removed from the electrode surface through physical abrasion, such as for example polishing. In this way, each CPE can be reused several times.

Screen-printed electrodes (SPEs) are also used in the field of analytical chemistry. SPEs comprise a flat body formed of a number of different layers of inks printed on a physical substrate. SPEs are simple, low cost, effective and reliable devices. For detection of magnetic electrochemical immunoassays, SPEs are used together with permanent magnets that are arranged below the working electrode for confining the magnetic particles.

For instance, document WO2014147049 discloses a method for magneto-electrochemical detection of an analyte in a sample. The method is carried out through the use of a device that includes screen-printed carbon, screen-printed gold or screen-printed platinum electrodes. A sample, magnetic particles modified with a bioreceptor, and a recognition element linked to an enzyme are incubated. The SPE combined with a magnet is then applied to the result of the incubation. An electrochemical substrate of the enzyme is added and the enzymatic reaction is allowed to proceed to obtain a compound with redox capability which is electrochemically measured on the electrode surface. The presence of the analyte is determined by evaluating the result of the previous step with respect to a reference.

Finally, some few works describe the utilization of thin-film planar electrodes for the electrochemical detection of bioassays performed at magnetic particles. Again, for detection each electrode is arranged above a permanent magnet. After detection, the magnet is either shielded or physically separated from the electrode, the particles are released, and the electrode can be treated appropriately for its reutilization.

One disadvantage of the devices described above is that the reuse of the electrodes after the analysis requires either extensive treatment of the CPE or the electrodes to be physically moved away from the magnets. Besides, the deposition of the solution covering the electrodes should be done very carefully since the electrodes could be moved during this process. Furthermore, the fact that such devices are often hydrophobic results in that the deposition of the solution is time consuming when trying to spread a drop of the solution through, for example, pipetting. Feeding higher solution volumes on the devices can facilitate this task but also increases the risk of spilling, with the drop potentially falling on the sides of the device and/or wetting the electrical contacts, resulting in that the process should be started again which undesirably results in a longer processing time.

On the other hand, magnetic separation racks are also known in the art. Magnetic separation racks are adapted to hold a number of tubes containing samples to be processed. The magnetic racks comprise a tube support and a magnetic support. The magnetic support includes permanent magnets attached/assembled thereto, arranged near the tube support for performing magnetic concentration/confinement of magnetic particles after incubation and/or washing steps. In this way, magnetic particles and any bound molecules can be physically separated from any other solution components, to be subsequently re-suspended in a solution of appropriate volume and composition. In said known devices, the release of the magnetic particles, for instance for their re-suspension, requires moving each container where the magnetic particles are contained to a new position, away from the magnets.

### Summary

Many of the above mentioned problems in prior art devices can be at least partially reduced through the device and the method for electrochemical detection with magnetic particles which will be described hereinbelow.

Although the present disclosure relates to a device and a method for electrochemical detection with magnetic particles, they are however not limited to these applications but they can be used in general for the performance and/or detection of magnetic assays. For example, a similar principle of action could be applied for the reversible confinement of magnetic particles into tubes, instead of onto electrodes, to facilitate the incubation and washing steps performed along the assay without having to relocate the tubes. Likewise, a similar principle of action could be exploited for the reversible confinement of the magnetic particles using appropriate containers or transducers for the optical detection of the bound targets, either in the vicinity of the particles or in a different position, such as upstream or downstream in a microfluidic channel or a capillary.

Concerning the magnetic affinity assay, besides using bioreceptors coupled to the magnetic particles, electrostatic capture of target molecules, such as drugs or neurotransmitters, employing particles with surface charges, or other types of molecular interactions, could be exploited for binding the target of interest.

As used herein, magnetic particles refer to particles, such as for example beads, responsive to a magnetic field. The magnetic field may be generated for example by magnets such as permanent magnets. The magnetic particles may be modified or have a surface with (bio)receptors, (bio)molecules or analytes of interest.

The magnetic particles may be modified with a single type of (bio)receptor, (bio)molecule or analyte, or with combinations of different forms thereof. For example, the magnetic particles may be modified simultaneously with antibodies against one analyte to detect such first analyte and with another analyte to detect the antibody against this second analyte, or they can be modified with both antibodies and aptamers, i.e. a different type of receptor, where the assay involves a single capture event per molecular target.

The magnetic particles may be modified with a single (bio)receptor, (bio)molecule or analyte, or combinations thereof (e.g., the magnetic particles may be modified simultaneously with two or more antibodies/bioreceptors that recognize different epitopes in the same analyte), where the assay consists of two or more events of molecular target capture. In this case, different levels of aggregation/agglutination of the magnetic particles are induced by the capture.

All or part of the magnetic particles may be modified with molecular markers (e.g., enzymes, electroactive molecules, nanoparticles, nanostructures, etc.). In this case, steric hindrance is produced by subsequent capture at least in part of the markers, affecting in some way the electrochemical detection.

The present electrochemical detection device generally comprises a base component, a cover component and one or more magnet supports.

As used herein, a base component refers to a part of the device at least a portion of which is capable of performing the function of supporting other parts of the device such as the magnet support.

As used herein, a cover component refers to a part of the device at least a portion of which is capable of performing the function of at least partially covering the base component.

In some cases, the present electrochemical detection device may have the base component and the cover component integral therewith. The present device can be thus formed by a single piece, with said single piece having at least one portion suitable for supporting other parts of the device, such as the magnet support, and with at least one portion suitable for at least partially covering the base component.

The base component comprises a body that is provided with at least one electrode receiving portion. Such electrode receiving portion(s) is/are adapted for supporting and retaining therein at least one electrode. The electrode(s) may be attached or assembled to the base component, for example glued or screwed to the electrode receiving portion, or it/they may be made integral with the base component; for example embedded therein. Examples of electrodes that can be used with the present device may be screen-printed electrodes (SPE) which have been shown to be highly effective in general for conducting electrochemical detection of magneto bioassays.

In line with the definitions given above for the cover component, the electrodes could act as the cover component as long as they are suitable for at least partially covering the base component.

When a separate cover component is provided, for example when a cover element as such is provided, the base component can be at least partially covered by the cover component mounted thereon and fixed thereto by any suitable means such as screws, pins, magnets, gluing, etc.

The cover component has at least one opening or window formed therein. Said at least one opening or window is adapted to make accessible an active area of the electrode or to give access thereto, allowing solutions to be easily and effectively deposited thereon. The openings in the cover component giving access to an active area of the electrode are configured to allow solutions such as a sample solution, a measuring solution or a washing solution, to be directly deposited in the vicinity of the electrodes, e.g. onto the surface of the electrodes. The size and/or the shape of the openings in the cover component are selected to properly confine a suitable volume of a solution for effectively conducting electrochemical detection. Accessing the electrodes through the openings in the cover component allows variable volumes of magnetic particles and/or solutions to be efficiently confined thereon without any overflow or spillage.

The openings in the cover component may be for example circular through holes conveniently sized as stated above for feeding magnetic particles in the vicinity of the electrodes, e.g. thereon as described, for electrochemical detection. Other shapes for the cover component openings may be also used.

The magnet support, whether a single or a plurality thereof are provided, comprises a support member adapted to carry at least one magnet, such as a permanent magnet, attached thereto. In some examples, the magnet support may have a linear and/or a polar array of magnets as required. The magnet(s) may be for example press-fitted into corresponding grooves formed in the magnet support. Other ways to attach or assemble the permanent magnet(s) to the magnet support such as, for example, through screws, gluing, etc. may be also possible.

The magnet support is mounted with respect to the base component such that the magnet support can be moved relative to the base component toward at least two different positions. In a first position of the magnet support, the magnet(s) act(s) on a corresponding electrode for confining the magnetic particles. In a second position of the magnet support, the magnet(s) do(es) not act on the electrode for allowing the magnetic particles to be released.

In this way, the movable magnet support with the permanent magnets together with the sets of electrodes act as a switchable electrode system. This advantageously allows the magnetic particles to be reversibly confined/released and electrodes to be reused. In addition, provision of a movable magnet support carrying multiple magnets together with a base component provided with multiple electrodes has the advantage that detection of a number of samples can be easily carried out in series or in parallel without moving the electrodes.

The movement of the magnet support with respect to the base component to said at least two different positions may be automated. For example, the magnet support may be power driven. Any suitable motor means may be provided for this purpose. Whether the magnet support is manual or power driven, the magnet support may be adapted to move relative to the base component according to a linear movement or according to an angular movement as required. In some examples, the magnet support could be moved relative to the base component according to a combination of linear and angular movements. In operation, the magnet support and the base component slide to each other according to a quick and short movement without affecting the other parts of the device.

It is envisaged that the magnet support may be adapted to move in a first direction according to a longitudinal axis of the base component. As used herein, 'according to a longitudinal axis of the base component' refers to a direction that at least substantially corresponds or is parallel to a longitudinal axis of the base component.

Alternatively or additionally, the magnet support may be adapted to move in a second direction perpendicular to said longitudinal axis of the base component.

Directions of the movement of the magnet support relative to the base component other than said first and second directions are also possible.

Irrespective of the directions of the movement of the magnet support, the magnet support may be adapted to move according to a continuous or a stepwise movement or a combination of both a continuous and stepwise movements as required. A stepwise movement could be advantageous in some cases to easily reach determined positions of the magnets relative to the electrodes as required.

In a further example of the electrochemical detection device, the magnet(s) may be positioned in the magnet support such that in said first position the magnet(s) is/are substantially aligned with a corresponding electrode which magnetic field causes the magnetic particles to be confined.

The magnet support may be adapted to apply a magnetic field in different ways. For example, the magnetic field may be applied by the magnets simultaneously and/or differentially and/or sequentially to at least some of the electrodes.

Electrical connections may be provided in the device for connecting the electrode(s) to external measuring equipment. Such electrical connections may for example be provided in the base portion or in the cover portion. The electrical connections are preferred to be arranged separated from the solution in order to avoid contact therebetween.

The present device has been shown to greatly facilitate electrochemical detection, for example in magnetic enzyme-linked immunosorbent assays (ELISA) which are widely used both in the market and in diagnostic laboratories. When said assays are carried out on the surface of electromagnetic particles they are referred to as magneto-immunoassays.

The present device may however be also efficiently employed in many other detection processes or analytical applications where magnetic particles are used such as, for example, detection and/or analytical processes in environmental monitoring, food safety, sports medicine, etc.

An effective yet simple and compact device is provided by means of which sequential detection of magneto-bioassays can be effectively conducted. The very simple structure of the present device results in short production times which gives rise to a significant reduction of costs. The present device also provides the above stated advantage that reuse of the electrodes is greatly facilitated due to their fixed arrangement in combination with the movable magnets. This advantageously extends the service life of the electrical connectors. A further advantage of the present device is that handling operations during washing of the magnetic particles are very simplified and shortened especially considering the fact that, for example, magneto-bioassays usually involve a large number of incubations and washing operations.

With the present device, magneto bioassays can be integrated in capillary flow detection formats in which case the provision of a magnet assembly that can be switched on and off allows the magnetic particles to be efficiently retained and released within a capillary or a microfluidic channel in one position, while conducting colorimetric, fluorescent, electrochemical, etc. assay detections in another position.

The present device may be adapted to use small sized electrodes. In some cases, the device may be sized such that the gap between electrodes corresponds to the size of multichannel pipettes. This advantageously allows handling to be facilitated since the device is not required to be disassembled for each washing process in order to remove the electrodes, the magnets and the connectors. Due to the integrated and compact configuration of the present device, washing processes can be automated using, for example, suitable ELISA plate washing equipment.

The above described device may be employed according to a specific method for performing electrochemical detection with magnetic particles. Many other different methods can of course be performed by the above described device.

The present electrochemical detection method comprises providing a sample to be tested and providing magnetic particles. The magnetic particles are deposited on at least one electrode in the above described device. When the magnetic particles have been deposited on at least one electrode of the device, the magnet support is moved (whether it is displaced, rotated, etc.) to a first position such that the magnetic field of the magnets in the magnet support acts on a corresponding electrode. This causes the magnetic particles to be effectively confined.

Electrochemical detection of the assay can be then conducted on the magnetic particles. For example, an affinity capture assay can be performed on the magnetic particles. However other assays are not ruled out, such as immunocapture, receptor-ligand interaction, ligand-aptamer interaction, electrostatic attraction, etc. In any case, all or part of the analyte of interest present in the sample is effectively confined on the surface of the magnetic particles.

Once the assay has been completed, the magnet support is moved (whether it is displaced, rotated, etc. as stated above) to a second position. In this second position, the magnets in the magnet support are moved away from the electrodes such that the magnetic field does not act on the electrodes. The magnetic field is thus switched off and the magnetic particles can be properly released.

The method may include further steps. For example, a washing step may be performed for washing the surface of the particles in order to remove molecules or components that may be absorbed thereon.

The assay may also include conducting an incubation with bioreceptors modified with enzyme markers, electroactive markers or any other type of markers that contribute to generate or amplify a signal generated, for example by latex particles, particles of conductive material or polymer particles capable of modifying the behaviour of the magnetic particle sediment confined on the electrode.

The method may further include a treatment of the electrodes prior to their use, such as washing, activation, electrical connection, etc.

Depending on the detection strategy it may be necessary to remove the supernatant, for example, by pipetting, through absorption using a vacuum trap, by applying an absorbing material, etc. A more suitable solution can be then added for performing electrochemical detection, such as a suitable electrolyte carrier, or a solution of certain pH, salinity, composition or temperature to dissolve or solubilise nanolabels or release the analyte or other components, or a certain enzyme substrate when detection is performed on an enzyme.

Depending on the assay and the detection strategy it may be necessary to remove again the supernatant, for example, by pipetting, through absorption using a vacuum trap, by applying an absorbing material, etc. Then a more suitable solution or enzymatic substrate can be added for a second round of electrochemical detection, for example when the assay includes performing detection of more than one analyte or parameter using two or more molecular markers.

It may be necessary to conduct a washing step for washing or conditioning the electrodes and/or the openings of the cover component of the device. This can be performed through the use of chemical agents such as solvents, detergents, saline solutions, acidic or basic solutions, etc. and by different means such as pipetting, bubbling, flow, etc. However, the electrode washing/conditioning step may be carried out through the use of physical means such as a swab, a brush, physical abrasion, a magnetic cleaner, ultrasonic cleaning, etc. Electrochemical means may be also used such as electrode electrochemical activation. A further electrode drying step may be also performed such as air drying, using an air, N₂ or argon stream.

Additional objects, advantages and features of examples of the present electrochemical detection device and method will become apparent to those skilled in the art upon examination of the description, or may be learned by practice thereof.

### BRIEF DESCRIPTION OF THE DRAWINGS

Examples of the present device and method will be described in the following with reference to the appended drawings.

In the drawings:
Figure 1 is an exploded perspective view that diagrammatically shows a first example of the present electrochemical detection device;
Figure 2 is a top plan view of the base component of the electrochemical detection device shown in figure 1 with the magnet support positioned in a first relative position where a magnetic field is applied to the electrodes;
Figure 3 is a top plan view of the base component shown in figure 2 with the magnet support positioned in a second relative position where no magnetic field is applied to the electrodes;
Figure 4 is an exploded perspective view that diagrammatically shows a second example of the present electrochemical detection device;
Figure 5 is a top plan view of the base component of the electrochemical detection device shown in figure 4 with magnet supports positioned in different relative positions to the base component;
Figure 6 is a general perspective view that diagrammatically shows a third example of the present electrochemical detection device;
Figure 7 is an enlarged detail part view of the example of the electrochemical detection device shown in figure 6 where its interior is partially shown;
Figure 8 is an elevational side view of the example of the electrochemical detection device shown in figures 6 and 7 with the magnet support positioned in a first relative angular position where a magnetic field is applied to the electrodes; and
Figure 9 is an elevational side view of the example of the electrochemical detection device in figure 8 but with the magnet support positioned in a second relative angular position where no magnetic field is applied to the electrodes.

### DETAILED DESCRIPTION OF EXAMPLES

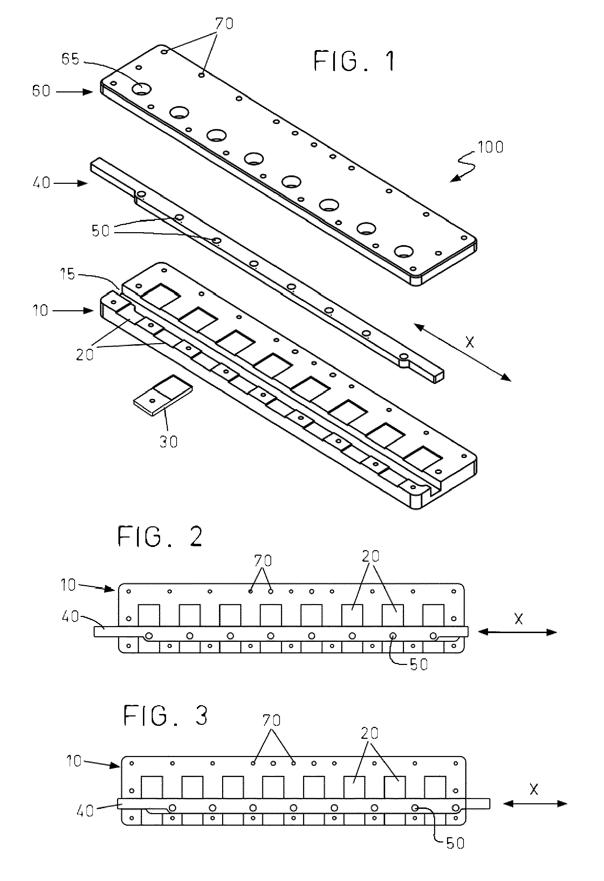

Figures 1-9 of the drawings depict three particular examples of the present electrochemical detection device. In said examples, the device has been indicated as a whole by reference numeral 100. The device 100 is intended mainly for electrochemical detection processes using magnetic particles. However, it is by no means limited to such application and it can be efficiently used, for example, to facilitate handling of magnetic bioassays based on the utilization of bioreceptors, electrostatic capture of molecules, such as drugs or neurotransmitters, employing particles with surface charges, or other types of molecular interactions, etc. Similarly, the same principle of action could be applied to other detection or transducing formats/strategies.

Magnetic particles, i.e. particles that are responsive to a magnetic field and modified or having (bio)receptors, (bio)molecules or analytes of interest on their surface are used for selective isolation, separation, purification and/or concentration processes. The magnetic particles are added to a sample such that they bind to selected target molecules present in the sample. They can be then isolated by applying a magnetic field.

In the three examples shown in figures 1-9 of the drawings, the electrochemical detection device 100 generally comprises a base component 10, a cover component 60 covering the base component 10 and one or more magnet supports 40 carrying a number of permanent magnets 50.

In the first and second examples of the device 100 shown in figures 1-5 of the drawings, the base component 10 and the cover component 60 are separate parts. However, the base component 10 should be interpreted within the meaning of the present disclosure as any part of the device 100 that is capable of performing the function of supporting other parts of the device such as the magnet support 40 as it will be described below. Similarly, the cover component 60 should be interpreted as any part of the device 100 that is capable of performing the function of at least partially covering the base component 10.

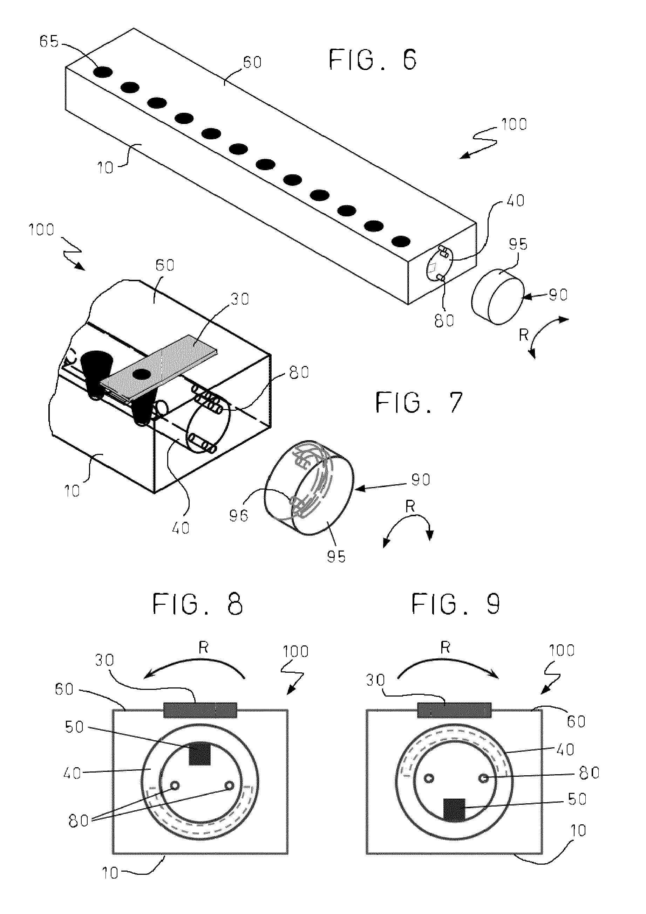

On the other hand, and such as it will be described further below, in the third example of the device 100 shown in figures 6-9 of the drawings, the cover component 60 is made integral with the base component 10.

In all of the examples of the electrochemical detection device 100 shown in the figures 1-9, the permanent magnets 50 are arranged and distributed as required, such as for example according to a linear array, a polar array, etc. Other configurations are not ruled out. The permanent magnets 50 may be attached or assembled to the magnet support 40 through a number of different ways such as press-fitted into grooves formed in the magnet support 40. The permanent magnets 50 may be also fastened with screws, pins, magnetic means, gluing, or any other means, or they may even be embedded in said grooves in the magnet support 40. A combination of means for attaching or assembling the magnets 50 to the magnet support 40 is also possible.

In the three examples of the device 100, the base component 10 comprises a body that is made, for example, of polymethyl methacrylate (PMMA). Other materials for the body of the base component 10 as well as for other parts of the electrochemical detection device 100 are not ruled out. The body of the base component 10, as well as most of the parts of the device, can be manufactured by using machining techniques. However, they could be also produced by other means, such as 3D printing, mould casting, etc. The body of the base component 10 is provided with a number of electrode receiving portions or pockets 20. The pockets 20 are so sized and shaped for receiving therein screen-printed electrodes (SPE) 30 retained therein. Each pocket 20 has one SPE 30 attached or assembled therein. However, a number of SPEs 30 may be attached or assembled to each pocket 20 in the base component 10. For the sake of clarity, only one SPE 30 has been diagrammatically depicted in figure 1 of the drawings. Types of electrodes other than SPEs may be of course used with the present electrochemical detection device 100 depending on the requirements for conducting electrochemical detection.

Sealing members, not shown, are also provided for properly sealing the electrodes 30 in order to prevent fluid leakages to the electrical connections. The sealing members may comprise 0.1 - 5 mm thick silicone gaskets, such as 0.5 - 1 mm thick silicone gaskets made through suitable cutting techniques. The silicone gaskets may have an acrylic adhesive layer on one side such that they are attached or assembled to the side of the base component 10 that is in direct contact with the electrodes 30. O-rings of different materials and other types of sealing members may be also used. Referring to the first example of the electrochemical detection device 100 shown in figures 1-3, the base component 10 includes eight pockets 20 for receiving corresponding eight electrodes 30. Other number of pockets 20 and electrodes 30 may of course be used according to the requirements. The base component 10 further includes a longitudinal groove or channel 15. The groove or channel 15 is conveniently sized to accommodate a laterally sliding magnet support 40. The magnet support 40 is adapted to carry eight permanent magnets 50 attached or assembled therein.

The magnet support 40 with the permanent magnets 50 can be moved according to a quick and short sliding movement in a first direction according to arrow X depicted in figures 1- 3. Direction X of the magnet support sliding lateral movement is parallel to the longitudinal axis of the base component 10. The sliding movement of the magnet support 40 with the permanent magnets 50 may be continuous or stepwise, or a combination of both continuous and stepwise as required. Such sliding movement does not affect other parts of the device 100. The magnet support 40 can thus reach a first position shown in figure 2 and a second position 3 shown in figure 3 of the drawings.

In the first position that can be reached by the magnet support 40 shown in figure 2, the magnet support 40 is positioned such that each permanent magnet 50 is below a corresponding electrode 30 substantially aligned thereto. In this first position of the magnet support 40 in figure 2, the permanent magnets 50 apply a magnetic field on the electrodes 30 such that the magnetic particles can be confined.

In the first position of the magnet support 40, the magnetic field is applied simultaneously to some or all of the electrodes 30. The magnetic field could be also applied differentially and/or sequentially to some or all of the electrodes 30.

In the second position that can be reached by the magnet support 40 shown in figure 3, the magnet support 40 is positioned such that each permanent magnet 50 is between two electrodes 30. In this second position of the magnet support 40 in figure 3, no magnetic field is applied to the electrodes 30 such that the magnetic particles can be released.

Thus, the first and second positions of the magnet support 40 causes the magnetic field to be switched on/off as required.

In the first example of the electrochemical detection device 100 shown in figure 1, the cover component 60 includes eight circular openings or windows 65 in line with the number of electrodes 30 and magnets 50. Other number, size and shapes for the windows 65 in the cover component 60 may of course be used according to the requirements. The windows 65 give access to the active area of the electrodes 30 when the cover component 60 is properly attached or assembled to the base component 10.

Solutions such as sample solutions, magnetic particles, additional reagents/(biocomponents), washing solutions and/or measuring solutions can be thus easily deposited on the electrodes 30 through the windows 65 in the cover component 60. This prevents solutions from overflowing while a suitable volume of solution can be suitably confined for effectively conducting electrochemical detection. Variable volumes of, for instance, magnetic particle containing solution can be efficiently confined without any overflow or spillage.

A number of holes 70 are formed along the perimeter of both the base component 10 and the cover component 60 of the device 100. The holes 70 are intended for receiving attaching screws (not shown) to attach or assemble both components 10, 60 to each other. Other attaching or assembling means could be also used alternatively or in addition such as pins, magnets, etc. As stated above, in cases such as in the third example of the electrochemical detection shown in figures 6-9 of the drawings, the cover component 60 may be made integral with the base component 10.

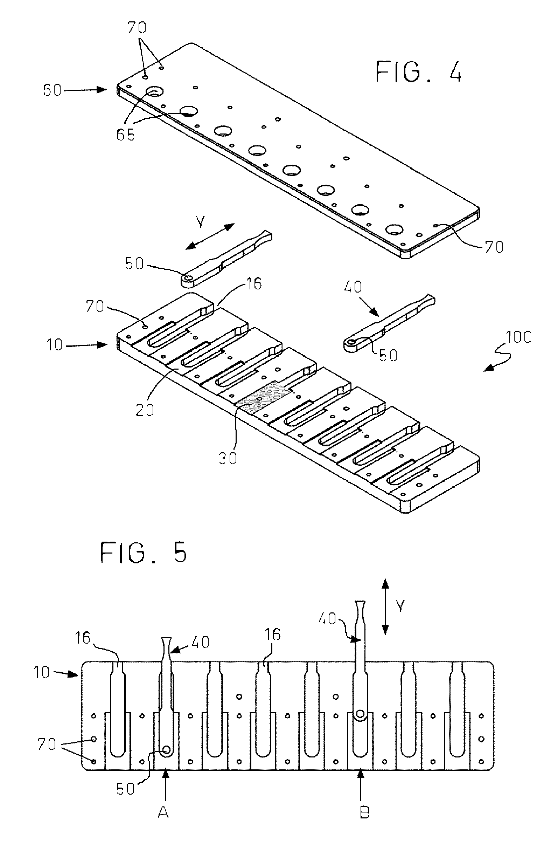

Referring now to the second example of the electrochemical detection device 100 shown in figures 4 and 5 of the drawings, the base component 10 includes eight pockets 20 for receiving eight screen-printed electrodes (SPE) 30. Other numbers and/or types of electrodes 30 may be of course used in this example, such as planar macroelectrodes, thin-film electrodes, and any other type of electrodes. For the sake of clarity, only one SPE 30 has been diagrammatically depicted in figure 4.

The base component 10 of the device 100 in this second example includes eight transverse grooves 16. The transverse grooves 16 are sized and shaped to accommodate front-to-back sliding magnet supports 40, each carrying permanent magnets 50. The magnet supports 40 can be moved with the permanent magnets 50 carried therein along such transverse grooves 16 in a second direction according to arrow Y depicted in figures 4 and 5. In the examples shown, the second direction Y is at least substantially perpendicular to the first direction X shown in figures 1- 3.

In use, the magnet supports 40 performs a quick and short sliding movement without affecting the other parts of the device 100. Such sliding movement of the magnet supports 40 may be continuous or stepwise, or a combination of both continuous and stepwise, as required. Said sliding movement allows a first position A and a second position B to be reached which will be described below.

Some of the magnet supports 40 may be moved along the second direction Y to reach the first position A while some may be moved along the second direction Y to reach the second position B as shown in figure 5. However, in other cases, the device 100 could be arranged such that all of the magnet supports 40 are moved at the same time along the second direction Y to reach the first position A or the second position B.

In the specific example shown in figure 5, the permanent magnets 50 in the first position A are positioned below an electrode 30, substantially in line therewith. This results in that a magnetic field is applied to electrode 30 in a way that the magnetic particles can be effectively confined. The permanent magnets 50 in the second position B are driven off from the electrode(s) 30. In this case, no magnetic field is applied to electrode 30 such that the magnetic particles can be released.

Therefore, positioning of the magnet support 40 in said first and second positions A, B causes the magnetic field to be switched on/off as required.

In the second example of the electrochemical detection device 100 shown in figures 4 and 5, the cover component 60 includes eight circular openings or windows 65, in line with the number of electrodes 30. Other number, size and shapes for the windows 65 are not ruled out. The windows 65 are adapted to give access to the active area of the electrodes 30 when the cover component 60 is properly attached or assembled to the base component 10.

A number of holes 70 are formed along the perimeter of both the base component 10 and the cover component 60 of the device 100. The holes 70 are intended for receiving attaching screws (not shown) to attach or assemble both components 10, 60 to each other. As in the first example described above with reference to figures 1-3, other different attaching or assembling means could be also used alternatively or in addition, such as pins, magnets, gluing, etc. In some cases, such as in the third example of the electrochemical detection shown in figures 6 and 7 of the drawings, the cover component 60 may be made integral with the base component 10 as it will be described below.

Referring to figures 6-9 of the drawings, a third example of the present electrochemical detection device 100 is shown. In this example, the cover component 60 is made integral with the base component 10. The base component 10 is adapted for receiving a number of screen-printed electrodes (SPE) 30, only one of which has been depicted in figure 7 for the sake of clarity. Other types of electrodes 30 may of course be used, such as, planar macroelectrodes, thin-film electrodes, and any other type of suitable electrodes.

In this case, a rotary magnet support 40 is provided. The rotary magnet support 40 is adapted for carrying a number of permanent magnets 50, such as twelve. The permanent magnets 50 can be viewed in the schematic elevational side views of figures 8 and 9 of the drawings. In this particular example, the magnet support 40 is mounted such that it can be rotated around the longitudinal axis of the base component 10, according to arrow R in figures 6-9. Rotation of the magnet support 40 allows the magnets 50 carried therein to reach at least two different angular positions relative to the base component 10 depending of the direction of rotation R. In the first angular position of the magnet support 40 shown in figure 8, the permanent magnets 50 are placed just below the electrodes 30. This results in that a magnetic field is applied to the electrodes 30 in a way that the magnetic particles are properly confined. In the second angular position of the magnet support 40 shown in figure 9, the permanent magnets 50 are positioned away from the electrode 30. In this case, no magnetic field is applied to the electrodes 30 in a way that the magnetic particles can be released.

The magnet support 40 has pins 80 protruding outward from one end of the magnet support 40. The protruding pins 80 act as a stop for limiting rotation of the magnet support 40 and for defining the first and second angular positions of the magnet support 40 shown in figures 8 and 9 of the drawings. An actuator terminal portion 90 located at one side of the device 100 is provided for appropriately and easily driving the magnet support 40 in rotation. The actuator terminal portion 90 shown in figures 6 y 7 comprises a cylindrical member 95 having corresponding complementary recesses 96. The recesses 96 are conveniently adapted for receiving the protruding pins 80 of the magnet support 40. Rotation of the actuator terminal portion 90 by the user according to arrow R in figures 6-9 results in rotation of the magnet support 40 with the permanent magnets 50 attached or assembled thereto causing the magnetic field to be switched on/off as required.

Therefore, in the examples shown in the drawings 1-9, the movable magnet support 40, together with the permanent magnets 50 and the sets of electrodes 30 act as a switchable electrode system. The magnetic particles can be thus reversibly confined and the electrodes 30 of the electrochemical detection device 100 can be advantageously reused for further processes.

The electrochemical detection device 100 can be used as follows. A sample to be tested is provided. Magnetic particles are deposited on one or more electrodes 30 through the openings in the cover component 60. The magnet support 40 is then displaced (examples 1 and 2) or rotated (example 3) to a first position such that the magnetic field from magnets 50 acts on a corresponding electrode 30. This causes the magnetic particles to be confined.

Then, electrochemical detection assay (receptor-ligand interaction, ligand-aptamer interaction, electrostatic attraction, etc.) is performed on the magnetic particles with all or part of the analyte of interest present in the sample being confined on the surface of the magnetic particles deposited onto the electrodes in the device 100. Once the assay has been completed, the magnet support 40 is displaced (examples 1 and 2) or rotated (example 3) to a second position such that the magnetic field of the magnets 50 does not act on a corresponding electrode 30. This allows the magnetic particles to be released.

A washing step may be performed at any time of the protocol for washing the surface of the particles to remove molecules or components that were weakly absorbed thereon. The electrodes 30 can be also washed after magnetic particle release and can be directly reused for a new detection cycle

An incubation step may be carried out with bioreceptors/ligands modified with enzyme markers, electroactive markers or any other type of markers that contribute to generate or amplify the signal generated, including for example latex particles, particles of conductive material or polymer particles that may modify the behaviour of the magnetic particle sediment confined on the electrode 30.

The method may further include a treatment step for treating the electrodes 30 prior to their use. The electrode treatment step may be for example washing, activation, electrical connection, etc.

Other steps than can be carried out depending on the detection strategy, which may include among others removal of supernatant, for example, by pipetting, through absorption using a vacuum trap, by using an absorbent material. Then a more suitable solution can be added for electrochemical detection such as a suitable electrolyte carrier, or a certain enzyme substrate when detection is performed on an enzyme, or a solution of certain pH, salinity, composition or temperature to dissolve or solubilise nanolabels or release the analyte or other components.

The supernatant may be required to be removed again, for example, by pipetting, through absorption using a vacuum trap, by using an absorbent material, etc. Then a more suitable solution or enzymatic substrate can be added for additional rounds of electrochemical detection, for example when the assay includes detection of more than one analyte or parameter using two or more molecular markers.

Finally, it might be necessary to perform a washing/conditioning step of the electrodes 30 and/or the openings or windows 65 of the cover component 60 of the device 100. This can be performed by means of chemical agents such as solvents, detergents, saline solutions, acidic or basic solutions, etc. and by different means such as pipetting, bubbling, flow, etc. However, the electrode washing/conditioning step may be carried out through physical means such as a swab, a brush, physical abrasion, a magnetic cleaner, ultrasonic cleaning, etc., electrochemical means such as electrode electrochemical activation. A further electrode drying step may be also performed such as air drying, using an air, N₂ or argon stream.

Although only a number of particular examples of the present electrochemical detection device and method have been disclosed herein, it will be understood by those skilled in the art that other alternative examples and/or uses and obvious modifications and equivalents thereof are possible. Furthermore, the present disclosure covers all possible combinations of the particular examples described.

For example, the base component 10 could be adapted for carrying different types of electrodes other than those disclosed and shown herein, for example in terms of sizes and/or shapes and/or geometries such as for example thin-film electrodes. It is also envisaged that different sets of electrodes may be provided in the same base component 10, for example.

The movement of the magnet support 40 with respect to the base component 10 to the different positions might be power driven. Power driven magnet supports might be automated e.g. using appropriate control means.

The device 100 shown in the examples in figures 1-9 could be used in many other applications. For example, in the third example, holes could be provided for holding Eppendorf tubes to perform incubations and washing operations of the magnetic particles without requiring the tubes to be relocated. The cover component 60 could be adapted to facilitate magnetic particle serial incubation while facilitating washing steps inside microtubes as they are not required to be physically displaced.

Although in some examples the magnet support 40 has been described as being capable of moving along a first direction X that is parallel to the longitudinal axis of the base component 10, and in other examples along a second direction according to arrow Y that is perpendicular to said longitudinal axis, directions of the movement of the magnet support 40 relative to the base component 10 other than said first and second directions X, Y are also possible.

The scope of the present disclosure is thus not limited by the particular examples disclosed, but should be determined only by a fair reading of the claims that follow.

Reference signs related to drawings and placed in parentheses in a claim, are solely for attempting to increase the intelligibility of the claim, and shall not be construed as limiting the scope of the claim.

## Claims

1. A device (100) for electrochemical detection with magnetic particles comprising:
- a base component (10) having at least one electrode receiving portion (20) for supporting and retaining therein at least one electrode (30);
- a cover component (60) for at least partially covering the base component (10) and having at least one opening (65) for feeding magnetic particles in the vicinity of the electrode (30) for electrochemical detection, the cover component (60) being adapted to confine a volume of a solution for conducting electrochemical detection; and
- at least one magnet support (40) having at least one magnet (50) and mounted with respect to the base component (10) such that the magnet support (40) can be moved relative to the base component (10) at least in a first position where the magnet (50) acts on a corresponding electrode (30) for confining the magnetic particles, and in a second position where the magnet (50) does not act on the electrode (30) for allowing the magnetic particles to be released.

2. The device (100) according to claim 1, wherein the cover component (60) is made integral with the base component (10).

3. The device (100) according to claim 1 or claim 2, wherein the magnet support (40) is adapted to move relative to the base component (10) according to a linear movement.

4. The device (100) according to any the preceding claims, wherein the magnet support (40) is adapted to move relative to the base component (10) according to an angular movement.

5. The device (100) according to any of the preceding claims, wherein the magnet support (40) is adapted to move in a first direction according to a longitudinal axis of the base component (10).

6. The device (100) according to any of the preceding claims, wherein the magnet support (40) is adapted to move in a second direction perpendicular to a longitudinal axis of the base component (10).

7. The device (100) according to any of the preceding claims, wherein the magnet support (40) is adapted to move according to a continuous movement.

8. The device (100) according to any of the preceding claims, wherein the magnet support (40) is adapted to move according to a stepwise movement.

9. The device (100) according to any of the preceding claims, wherein the magnet (50) is positioned in the magnet support (40) such that in the first position the magnet (50) is substantially aligned with a corresponding electrode (30) which magnetic field causes the magnetic particles to be confined.

10. The device (100) according to any of the preceding claims, wherein the magnet support (40) has a linear array of magnets (50).

11. The device (100) according to any of the preceding claims, wherein the magnet support (40) has a polar array of magnets (50).

12. The device (100) according to any of the preceding claims, wherein the magnet support (40) is adapted to apply a magnetic field, in at least one of simultaneous, differential and sequential way, to at least some of the electrodes (30).

13. The device (100) according to any of the preceding claims, wherein it includes electrical connections for connecting the electrode (30) to external measuring equipment.

14. A method for electrochemical detection with magnetic particles employing an electrochemical detection device according to any of the preceding claims, the method further comprising:
- providing a sample to be tested;
- depositing magnetic particles on at least one electrode (30) in the device;
- moving the magnet support (40) to a first position such that a magnet (50) acts on a corresponding electrode (30) for confining the magnetic particles; and
- conducting the electrochemical detection of the assay such that at least a portion of an analyte in the sample being tested is confined on the surface of the magnetic particles.

15. The method of claim 14, wherein it further comprises moving the magnet support (40) to a second position such that the magnet (50) does not act on the electrode (30) for allowing the magnetic particles to be released.
